# EUROPEAN PATENT SPECIFICATION

(11) **EP 2 123 670 B1**
(45) Date of publication and mention of the grant of the patent: **23.08.2017**
(21) Application number: 08156740.6
(22) Date of filing: 22.05.2008
(51) Int. Cl.: C07K 14/745, C07K 14/81, C07K 14/435

(54) **Anticoagulant polypeptide**
Koagulationshemmendes Polypeptid
Polypeptide anticoagulant

(43) Date of publication of application: 25.11.2009
(73) Proprietor: Bioxodes SA, 6900 Marche-en-Famenne (BE)
(72) Inventor: Godfroid, Edmond, 1120 Bruxelles (BE); Decrem, Yves, 1380 Ohain (BE); Vanhamme, Luc, 1490 Court-Saint-Etienne (BE)
(74) Representative: Bonneton, Manon

(56) References cited:
- WO-A-00/77198
- US-B2- 6 794 166
- LEBOULLE GERARD ET AL: "Isolation of Ixodes ricinus salivary gland mRNA encoding factors induced during blood feeding" AMERICAN JOURNAL OF TROPICAL MEDICINE & HYGIENE, LAWRENCE, KS, US, vol. 66, no. 3, 1 March 2002 (2002-03-01), pages 225-233, XP002314131 ISSN: 0002-9637
- PETERSEN LARS C ET AL: "Inhibitory properties of separate recombinant Kunitz-type-protease-inhibitor domains from tissue-factor-pathway inhibitor" EUROPEAN JOURNAL OF BIOCHEMISTRY, vol. 235, no. 1-2, 1996, pages 310-316, XP009104389 ISSN: 0014-2956
- BAUGH ROBERT J ET AL: "Regulation of extrinsic pathway factor Xa formation by tissue factor pathway inhibitor" JOURNAL OF BIOLOGICAL CHEMISTRY, vol. 273, no. 8, 20 February 1998 (1998-02-20), pages 4378-4386, XP009104390 ISSN: 0021-9258

### SEQUENCE LISTING

<110> universite Libre de Bruxelles
<120> Anticoagulant polypeptide
<130> Ir-CPI
<160> 4
<170> PatentIn version 3.3
<210> 1
   <211> 204
   <212> DNA
   <213> Ixodes ricinus
<220>
   <221> CDS
   <222> (1)..(204)
<400> 1
   gcc aac cac aaa ggt aga ggg cgg cct gcg aag tgt aaa ctt cct ccg       48
   Ala Asn His Lys Gly Arg Gly Arg Pro Ala Lys Cys Lys Leu Pro Pro
   1               5                   10                  15
   gac gac gga cca tgc aga gca cga att ccg agt tac tac ttt gat aga       96
   Asp Asp Gly Pro Cys Arg Ala Arg Ile Pro Ser Tyr Tyr Phe Asp Arg
               20                  25                  30
   aaa acc aaa acg tgc aag gag ttt atg tat ggc gga tgc gaa gga aac      144
   Lys Thr Lys Thr Cys Lys Glu Phe Met Tyr Gly Gly Cys Glu Gly Asn
           35                  40                  45
   gaa aac aat ttt gaa aac ata act acg tgc caa gag gaa tgc aga gca      192
   Glu Asn Asn Phe Glu Asn Ile Thr Thr Cys Gln Glu Glu Cys Arg Ala
       50                  55                  60
   aaa aaa gtc taa                                                      204
   Lys Lys Val
   65
<210> 2
   <211> 67
   <212> PRT
   <213> Ixodes ricinus
<400> 2
   Ala Asn His Lys Gly Arg Gly Arg Pro Ala Lys Cys Lys Leu Pro Pro
   1               5                   10                  15
   Asp Asp Gly Pro Cys Arg Ala Arg Ile Pro Ser Tyr Tyr Phe Asp Arg
               20                  25                  30
   Lys Thr Lys Thr Cys Lys Glu Phe Met Tyr Gly Gly Cys Glu Gly Asn
           35                  40                  45
   Glu Asn Asn Phe Glu Asn Ile Thr Thr Cys Gln Glu Glu Cys Arg Ala
       50                  55                  60
   Lys Lys Val
                         65
<210> 3
   <211> 33
   <212> DNA
   <213> Artificial sequence
<220>
   <223> Forward primer Ir-CPI
<400> 3
   cgcggatccg cggccaacca caaaggtaga ggg 33
<210> 4
   <211> 37
   <212> DNA
   <213> Artificial sequence
<220>
   <223> Reverse primer Ir-CPI
<400> 4
   ccgctcgagc ggttagactt tttttgctct gcattcc 37

## Description

### FIELD OF THE INVENTION

The present invention is in the field of pharmacy and is related to an antithrombotic polypeptide isolated from *I. ricinus.*

### BACKGROUND OF THE INVENTION

### Coagulation Pathways mechanism

Coagulation in mammalian plasma involves a large number of proteins acting in a cascade and leading to the generation of thrombin. This enzyme then converts fibrinogen into fibrin. According to current models of haemostasis, blood coagulation starts immediately after damage to the vascular endothelium. This exposes blood to subendothelial structures and components. The extrinsic coagulation pathway is therefore triggered by the binding of tissue factor produced by subendothelial cells to plasma factor VII. The resulting complex initiates the coagulation cascade, activates factor IX, which in turn activates factor X and ultimately leads to thrombin generation. Propagation of clotting involves several positive feedback mechanisms. For example, thrombin initiates the intrinsic coagulation pathway: it activates factor XI which activates additional factor IX, therefore amplifying the coagulation process.

Factor IX may also be activated *in vitro* by the contact phase system, the *in vivo* relevance of which is still controversial. Contact phase proteins include the zymogens factor XII, factor XI and prekallikrein, as well as the cofactor high molecular weight kininogen (HMWK). Factor XII undergoes autoactivation when bound to polyanionic surfaces, generating activated factor XII by a conformation change. Activated factor XII then converts prekallikrein into kallikrein by cleavage of a single peptide bond. Once small amounts of kallikrein are formed, they catalyze the conversion of surface-bound factor XII into factor XIIa leading to strong positive feedback on the system. During this process, factor XII is activated by a succession of proteolysis steps leading to the production of a series of different active enzymes: factor XIIa, active α-factor XIIa, and factor XIIf (Dunn, 1982).

*In vitro*, the contact phase system initiates the intrinsic coagulation pathway by cleavage of factor XI into activated factor XI (factor XIa) by α-factor XIIa. Factor XIIf may also activate factor VII, the proenzyme initiating the extrinsic coagulation pathway, dependent on tissue factor (TF).

It later became clear that activation of the contact-phase system is critically involved in proteolytic mechanisms distinct from coagulation, namely the kallikrein-kinin, complement, fibrinolytic, and renin-angiotensin systems. It therefore plays an essential role in fibrinolysis as it leads to the activation of plasmin and pro-urokinase by a mechanism that is still incompletely understood. It is also involved in the classic and alternative complement activation pathways: factor XIIf, kallikrein and plasmin are responsible for activation of the C1r and C1s subunits of the first complement component as well as factor B. Moreover, kallikrein is an activator of prorenin and is responsible for kinin formation.

The search for new drugs preventing thromboembolism is a major challenge in medicine. In practice, this involves identifying drugs capable of preventing thrombus formation without increasing the risk of haemorrhage. For the past forty years, anticoagulant treatment has been dominated by two classes of agents: heparins and antivitamins K. Heparins accelerate the inhibitory action of antithrombin on some activated coagulation factors (specifically thrombin and Xa factor) by indirect inhibition of these factors and are only active when administered parenterally. The latter prevents the final synthesis of four coagulation factors (prothrombin and factors VII, IX and X). These compounds require careful laboratory testing in order to guarantee sufficient antithrombotic effectiveness while avoiding the risk of haemorrhage.

This disadvantage together with the relatively narrow therapeutic margin of these drugs has considerably stimulated research into other types of agent. The choice of anticoagulant therapy either for venous thromboembolism or arterial thrombosis (myocardial infarction or stroke) is based on how well a drug inhibits thrombosis and its haemorrhagic side effects. The discovery that factor XII deficiency protects against thrombosis without causing spontaneous bleeding makes factor XII a unique and ideal target for drug design (Renne, 2007).

### Tick blood feeding

Ticks are obligate blood feeding arachnids. They infest many species of mammals, birds, reptiles and amphibians worldwide. They are the vectors of protozoan, bacterial and viral pathogens of prime medical and veterinary importance.

Long blood meal by ticks is only possible because these parasites have developed ways to circumvent host defense mechanisms including
i) haemostasis (coagulation, platelet aggregation and vasoconstriction),
(ii) inflammatory response, and
(iii) innate and adaptive immunity.

Many blood-sucking ectoparasites synthesize substances to thwart the defense mechanisms of the hosts on which they feed. Ticks in particular, produce salivary substances capable of modulating the host immune responses and maintain blood in a sufficiently fluid state to effectively acquire and digest their blood meal. A study comparing the sequences of coagulation inhibitors secreted by ticks showed that inhibitors from hard and soft ticks had different origins, indicating that this adaptation occurred independently in these two families of blood feeders after their evolutionary divergence. Haemostasis involves a network of factors organized in different pathways which can be activated independently. Ticks are therefore confronted with a redundant system and must simultaneously block different steps to obtain effective anti-haemostasis. This has been achieved during the long tick/host co-evolution as ticks produce a variety of compounds with anti-haemostatic activity.

When ticks take a blood meal, the action of the chelicerae and insertion of the hypostome into the host skin damages the epidermis and dermis, ruptures local blood vessels and thereby activates both the extrinsic and contact phase pathways. Patent application WO00/77198 describes cDNA and protein sequences synthesized by the salivary gland of *Ixodes ricinus*, including some bearing putative anticoagulant properties. These comparisons show that one of the disclosed polypeptide, "SEQ_7", is highly homologous to the human Tissue Factor Pathway Inhibitor (TFPI). TFPI is an inhibitor of serine proteases having 3 tandemly arranged Kunitz-type-protease-inhibitor (KPI) domains. Each of these units or motifs has a particular affinity for different types of proteases. The first and second KPI domains are responsible for the respective inhibition of VIIa and Xa coagulation factors. The third KPI domain apparently has no inhibitory activity.

It should be noted that the corresponding polypeptide sequence of "SEQ_7" cDNA clone is homologous to the region of the first KPI domain of TFPI and that the KPI is perfectly kept therein. This homology suggests that the "SEQ_7" protein should be a potential factor VIIa inhibitor and / or a factor X inhibitor and / or an inhibitor of the tissue factor pathway.

Other examples of tick-induced anti-haemostatic activity include apyrase, which hydrolyzes ADP (*Ornithodoros moubata*; *Ornithodoros savignyi*) ; moubatin, an *O*. *moubata* collagen inhibitor that interferes with platelet aggregation; TAP (tick anticoagulant peptide), isolated from the saliva of *O*. *moubata*, a specific fXa inhibitor; Ixolaris (*Ixodes scapularis*), which binds to fXa and prevents it from activating tissue factor complex - fVIIa, and Penthalaris, a protein with 5 Kunitz domains, inhibiting the activation of fX by tissue factor complex - fVIIa, by binding to fX or to fXa also isolated in *I. scapularis* (Francischetti, 2004).

### Contact phase inhibition

Few inhibitors acting on the contact phase pathway have so far been discovered. Haemaphysalin is an inhibitor of the kallikrein-kinin system discovered in *Haemaphysalis longicornis.* This molecule binds via its C terminal domain to the cell binding domains of high molecular weight kininogen and also fXIIa, thereby preventing the activation stages of compounds of the contact system (Kato, 2005).

### SUMMARY OF THE INVENTION

The present invention is related to a polypeptide, here named Ir-CPI, obtained from tick salivary glands and presenting more than about 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % sequence identity with SEQ ID.NO.2.

The present invention is also related to the polypeptide having the sequence SEQ.ID.NO.2.

Specifically, the invention provides in one aspect a pharmaceutical composition as defined in claim 1 and, in another aspect, a polypeptide as defined in the claims. The invention includes a pharmaceutical composition comprising an adequate pharmaceutical carrier and the polypeptide as defined in the claims. The scope of the invention, therefore, is defined by the claims and, in relation to the scope of the invention, the following disclosure is subject to the claims.

The polypeptide(s) according to the invention may be modified by, or linked to, at least one substitution group preferably selected from the group consisting of amide, acetyl, phosphoryl and/or glycosyl groups. Moreover, this polypeptide(s) may take the form of a "mature" protein. They may also be part of larger protein(s) or part of a fusion protein.

Another object of the present invention concerns variants of the polypeptide of the present invention. Preferably, said variants vary from the referent by conservative amino acid substitutions. Preferably, at least one residue is substituted in said variant with another residue of similar characteristics. Advantageously, the substitutions in said variant are among Ala, Val, Leu and Ile; among Ser and Thr, among the acidic residues Asp and Glu; among Asn and Gln; among the basic residues Lys and Arg; or among aromatic residues Phe and Tyr.

Preferably, in the variant(s) of the present invention, several amino acids are substituted, deleted or added in any combination, as defined in the claims. According to the disclosure and subject to the claims, preferably, 5 to 10, more preferably 1 to 5, more preferably 1 to 2 amino acids are substituted, deleted or added in any combination, in this variant(s).

This variant(s) may be a naturally occurring allelic variant(s) of an *Ixodes ricinus* Ir-CPI (SEQ.ID.NO.2).

The present invention is also related to a polynucleotide (SEQ.ID.NO.1) encoding the polypeptide(s) of the present invention. The present invention is also related to a vector comprising at least one element selected from the group consisting of the polynucleotide and the polypeptide according to the present invention.

Still another object of the present invention concerns a cell comprising the recombinant vector according to the invention.

As already stated, the present invention in one aspect is related to a pharmaceutical composition consisting of an adequate pharmaceutical carrier or diluent and an effective amount of the polypeptide, the polynucleotide being defined in the claims, preferably for a treatment and / or a prevention of thrombosis and / or cardiovascular and / or neurological diseases, preferably through the inhibition of the reciprocal activation of factor XII, prekallikrein and factor XI in human plasma, preferably without disturbing the clotting balance (*in vivo* thrombus formation without impairing haemostasis, *i.e.* the inhibition of intrinsic coagulation pathway and, to a lesser extent the extrinsic coagulation pathway and fibrinolysis *in vitro* and/or *in vivo*).

Advantageously, the pharmaceutical composition of the present invention, while preventing and/or curing thrombosis, neither inhibits activities of activated Factor X and/ or Factor VII nor reduce their content.

In addition, the pharmaceutical composition of the present invention, while preventing and/or curing thrombosis, does not affect blood clotting of the treated mammal subject (including a human patient).

Furthermore, the pharmaceutical composition of the present invention, advantageously and selectively inhibits the activity and/or content of Factor XIIa, Factor XIa, plasmin and kallikrein, agents of the contact phase.

The pharmaceutical composition of the invention is used to treat a disease selected from the group consisting of thromboembolism, stroke, myocardial infarction, cerebrovascular diseases, cerebral ischemia, pulmonary embolism, renal vein thrombosis and hepatic vein thrombosis.

The polynucleotides and polypeptides of the present invention may be employed as research reagents and materials for the development of treatments and diagnostics tools specific to animal and human disease.

The present invention will be described hereafter in reference to the enclosed figures presented as non-limiting examples of the present invention.

### DETAILED DESCRIPTION OF THE INVENTION

### Short description of the figures

**Figure 1** represents *in vitro* anticoagulant activity of Ir-CPI.
**Figure 2** represents effect of Ir-CPI on the thrombin activity profile during the coagulation process induced either by intrinsic or extrinsic coagulation pathway.
**Figure 3** represents inhibitory effect of Ir-CPI on generation of factor XIIa, factor XIa and kallikrein in human plasma.
**Figure 4** represents inhibitory effect of Ir-CPI on reconstituted systems.
**Figure 5** represents sensorgrams for interactions between coagulation factors and immobilized Ir-CPI measured by surface plasmon resonance.
**Figure 6** represents effect of Ir-CPI on stasis-induced venous thrombosis in rats.
**Figure 7** represents effect of Ir-CPI on venous thrombosis in mice.

### EXAMPLES

### Example 1

SEQ.ID.NO.1, the nucleotide sequence encoding the polypeptide according to the invention (SEQ.ID.NO.2, also hereafter referred to as Ir-CPI), was cloned in the expression vector pGEX-6P-1 in-frame with the coding sequence of glutathione S-transferase and expressed in bacteria. Affinity purification followed by cleavage with PreScission protease and further fast protein liquid chromatography yielded pure recombinant protein. Ir-CPI is about 20-fold smaller than an antibody, making said Ir-CPI protein very suitable as therapeutics, even where the target is difficult to reach.

### Example 2: Recombinant Ir-CPI prolongs activated partial thromboplastin (aPTT), prothrombin (Pt) and fibrinolysis times

Increasing amounts of Ir-CPI were incubated with human plasma for 2 min to test its effect on global haemostasis *in vitro.*

The recombinant protein had no effect on PFA closure time or platelet aggregation with whole blood. This surprisingly suggested that Ir-CPI did not interfere with primary haemostasis.

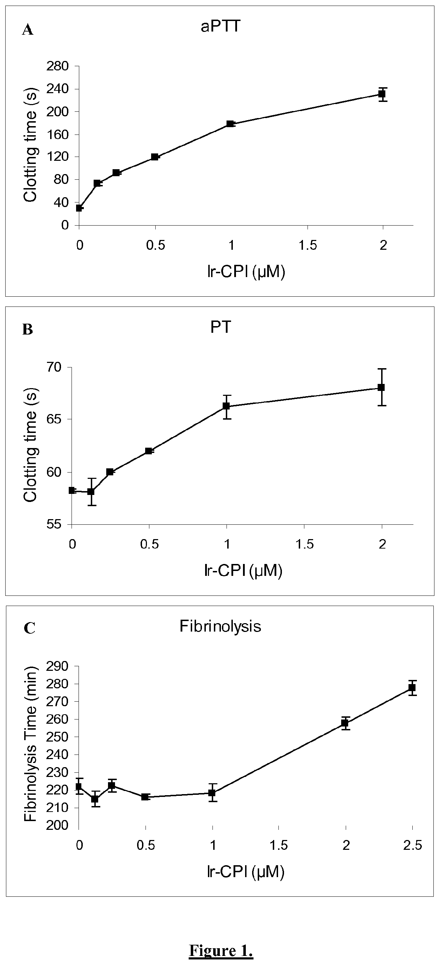

The anticoagulant activity of Ir-CPI was then assessed using four routine tests of plasma clotting time. Thrombin and RVV times remained unchanged, surprisingly evidencing that Ir-CPI did not interfere with fibrinogen cleavage or factor X activation. However advantageously, recombinant Ir-CPI prolonged aPTT (7.7 times at 2 µM) and PT (1.2 times at 2 µM) in a dose-dependent manner (Figures 1A and 1B), showing interference with the intrinsic pathway and, to a much lower extent, the extrinsic pathway (each point represents the mean ± SD of three independent determinations).

Finally, the activity of Ir-CPI was investigated on fibrinolysis. The results showed that the fibrinolysis time was slightly increased by 1.2 times in the presence of Ir-CPI at 2 µM (Figure 1C). All these experiments were also performed with GST used as a negative control. No interference was observed with none of the tests.

### Example 3: Ir-CPI inhibits intrinsic pathway-induced thrombin generation

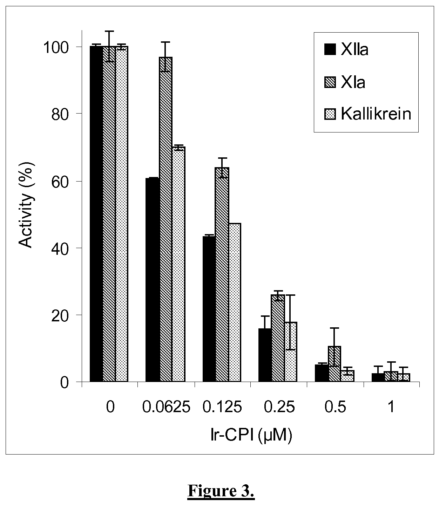

As thrombin is the key player in clot formation, the effects of Ir-CPI were investigated on its activity during the coagulation process. On the one hand, the intrinsic coagulation pathway was induced using a mixture of ellagic acid and phospholipids as triggers. The addition of recombinant Ir-CPI to the assay caused a dose-dependent prolongation of the lag time and a dose-dependent decrease in the peak concentration of active thrombin (Cmax) compared to the control curve (*i.e.* without inhibitor) (Figure 3A). Results are presented as the mean of three experiments.

At a final Ir-CPI concentration of 9.1 µM, the lag time was prolonged 3.6-fold compared to the control curve. Regarding Cmax, the effect was maximal at 0.7 µM and did not increase at higher concentrations (2.2 µM; 6.6 µM; 9.1 µM). At a concentration of 0.7 µM, the Cmax was reduced by 37% and the lag time was prolonged 2.7-fold.

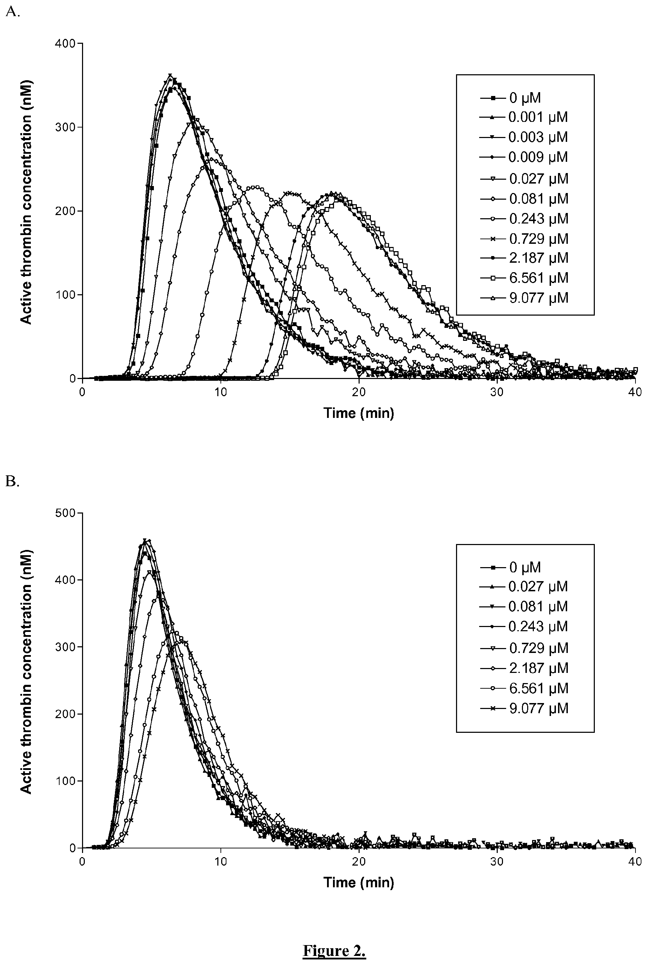

On the other hand, when the coagulation cascade was triggered by the extrinsic pathway (5 pM of tissue factor (TF) and 4 µM of phospholipids (PL)), a slight dose-dependent decrease in Cmax and a dose-dependent prolongation of the lag time were observed (Figure 2B). At a final Ir-CPI concentration of 9.1 µM, the Cmax was reduced by 30% and the lag time was prolonged 1.6-fold. Similar results were obtained when a lower concentration of TF (1 pM) and 4 µM PL were used as triggers.

These results confirm that Ir-CPI is a potent inhibitor of thrombin generation induced by the intrinsic pathway and, advantageously, to a much lower extent by the extrinsic pathway.

### Example 4: Ir-CPI inhibits the activation of contact system factors

In order to determine the target(s) of Ir-CPI, its effect on seven procoagulant active serine proteases (namely; kallikrein, factor XIIa, XIa, IXa, Xa, IIa and VIIa) and two fibrinolytic serine proteases (t-PA and plasmin) was measured in amidolytic tests using specific substrates for each of these serine proteases. Surprisingly, these assays failed to reveal any effect of the Ir-CPI protein on the amidolytic activity of these factors, evidencing an inhibition by steric hindrance, rather than by binding to the active site of the target protease.

The capacity of Ir-CPI protein to inhibit the activation of these factors was then analyzed on human plasma contact phase factors by incubating human plasma with Ir-CPI and then adding a contact phase activator. The activation of contact factors (factors XII, XI and kallikrein) was evaluated by using a specific substrate for each factor. The results showed that Ir-CPI inhibited the generation of the active form of these three factors in a dose-dependent manner (Figure 3).

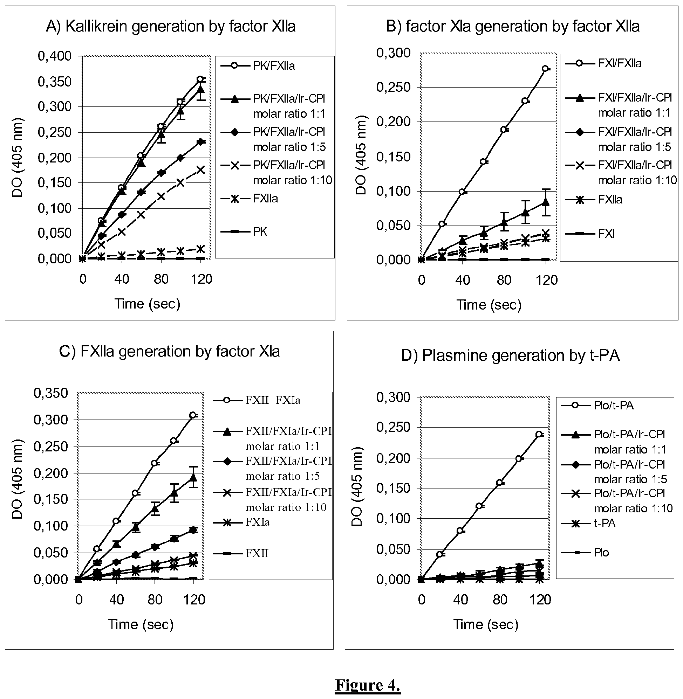

The effect of Ir-CPI on contact phase factors was further dissected and examined in reconstituted systems using purified factors and their specific chromogenic substrates. The activation of a non-activated factor by an activated factor, in the presence or absence of Ir-CPI was analyzed in each experiment. Results are presented as the mean ± SD of three determinations. In each of these assays, the inventors compared the activation of a non-activated factor (zymogen) by an activated factor, in the presence or absence of Ir-CPI. The results showed that Ir-CPI inhibited the activation of prekallikrein into kallikrein by factor XIIa, activation of factor XI into factor XIa by factor XIIa and activation of factor XII into factor XIIa by factor XIa (Figures 4A-C).

On the contrary, Ir-CPI failed to inhibit the activation of factor XII into factor XIIa by kallikrein or the activation of factor X into factor Xa by tissue factor/factor VIIa complex. However, it did inhibit the activation of plasminogen into plasmin by t-PA (Figure 4D).

The results of these experiments advantageously show that Ir-CPI only impacts the activation of factors participating in the contact phase of coagulation.

### Example 5: Ir-CPI binds to factor XIa, fXIIa, kallikrein and plasmin

The inventors then searched whether the inhibitory activity of Ir-CPI involved a direct interaction with one or more blood coagulation factors by evaluating the capacity of Ir-CPI to bind to coagulation or fibrinolysis (co)factors by surface plasmon resonance. In practice, Ir-CPI was immobilized on the surface of a CM5 sensor chip at a level of 1500 resonance units (RUs). Contact factors to be analysed (100 nM final concentration) were injected at a flow rate of 70 µl/min in HBS buffer, and association was monitored for 84 s. After return to buffer flow, dissociation was monitored for 150 s. The sensor chip surface was regenerated by a pulse injection of 25 mM NaOH after each experiment. The global equilibrium constant, K = kₐ₁kₐ₂/k_{d1}k_{d2}.

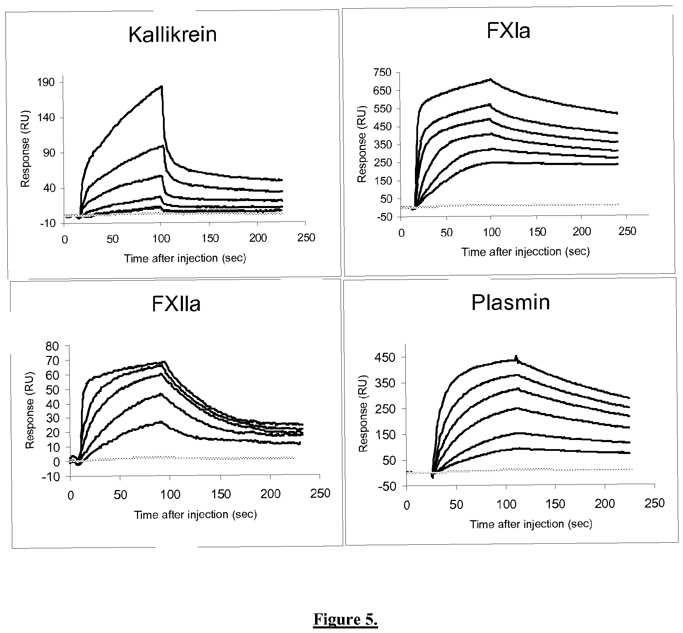

The assays demonstrated a specific interaction between Ir-CPI and four factors: fXIIa, fXIa, plasmin and kallikrein (Figure 5). Surprisingly, no interaction was observed with any of the other (co)factors tested (prekallikrein, HMWK, fXII, fXI, fIX, fIXa, fX, fXa, thrombin, fVIIa, t-PA and plasminogen).

In addition, the kinetics of interaction between Ir-CPI and the four targeted factors (XIIa, XIa, plasmin and kallikrein) were measured after immobilization of Ir-CPI. In assays designed to determine the binding kinetics, the quantity of immobilized Ir-CPI was deliberately kept at a low level (approximately 200 RU) in order to avoid problems due to limitation of the reaction rate by a mass-transport effect. The initial binding rate was shown to be independent of flow variations by linear regression measurements at the start of kinetics with injections of analytes at increasing flows from 30 to 70 µl/min. This confirmed that there was no limitation of the reaction.

Interaction kinetics were determined for each analyte at 6 different concentrations (from 5 nM to 300 nM). The data were processed with the BIA evaluation software and the best fits obtained with a two-state binding model which implies the binding of the plasma protein to immobilized Ir-CPI in a 1/1 ratio followed by a reversible conformation change as in scheme 1 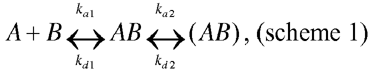 where A is the plasma protein, B the immobilized Ir-CPI, and (AB) is the complex form after conformational change in the initial complex AB. In this model, kₐ₁ and k_{d1} are the association and dissociation rate constants for the formation of AB and kₐ₂ and k_{d2} the forward and backward rate constants for the AB↔(AB) conformation change. The values of these rate constants are shown in Table 1.

**TABLE 1.**

| **Kinetic constants for Ir-CPI interactions with Kallikrein, FXIa, FXIIa and Plasmin** | | | | | |
|---|---|---|---|---|---|
| | kₐ₁ (1/Ms) | k_{d1} (1/s) | kₐ₂ (1/s) | k_{d2} (1/s) | K (1/M) |
| Kallikrein | 3.49±0.54 10⁴ | 5.32±0.92 10⁻² | 8.34±0.07 10⁻³ | 1.42+0.11 10⁻³ | 4.51±0.19 10⁶ |
| FXIa | 2.31±0.59 10⁶ | 1.02±0.51 10⁻¹ | 2.98±0.29 10⁻² | 2.32±0.28 10⁻³ | 3.83±2.37 10⁸ |
| FXIIa | 2.19±0.06 10⁵ | 9.46±0.04 10⁻² | 6.55±0.66 10⁻¹ | 1.05±0.04 10⁻¹ | 1.68±1.10 10⁸ |
| Plasmin | 9.00±0.39 10⁵ | 5.35±1.33 10⁻³ | 5.82±0.99 10⁻³ | 6.14±0.96 10⁻³ | 3.30±0.57 10⁸ |

These results indicate that Ir-CPI specifically binds to factor XIIa, XIa, kallikrein and plasmin, but not to their zymogenic forms. It should however be noted that the values in Table 1 are only indicative of the orders of magnitude. Indeed, large variations of the constants are observed when the association curves (0 to 84 s in Figure 5) are analyzed individually. Similarly, when the dissociation curves are fitted to a sum of two exponentials (y = yf + Ae-k't + Be-k"t, where t is the time, and k' and k" complex functions of kₐ₁, kₐ₂, k_{d1} and k_{d2}), the best fits are obtained with non-zero values of yf. This indicates that, with two exponentials, dissociation is not complete when time becomes large, which suggests a third, irreversible or very slowly reversible step after the AB↔(AB) conformation change. This "irreversible component" represents 20-40 % for Kallikrein, 53-60 % for fXIa, 10-24 % for fXIIa and 19-27 % for plasmin. Note that, surprisingly and in contrast to the 3 other proteins, plasmin also binds to immobilized GST but in this case, dissociation is complete within less than 200 s.

### Example 6: Ir-CPI interferes with thrombus formation in animal models of stasis-induced venous thrombosis

Before testing the antithrombotic action of Ir-CPI had an *in vivo,* the inventors first evaluated the half-life of Ir-CPI *in vivo.* A semi-quantitative estimate of Ir-CPI pharmacokinetics was obtained using 125I-Ir-CPI. The results showed that plasma 125I-Ir-CPI concentrations were about 40.8 % ± 9.9 % of the maximum value 20 h after intravenous administration of the recombinant protein. The effect of Ir-CPI on thrombus formation was then assessed by using two thrombosis models in experimental animals.

In the first model, in rats, venous thrombosis was induced by stasis after vessel ligation and activation of thrombosis by severe endothelial damage and vessel occlusion with ferric chloride. Ir-CPI at the indicated doses was administered i.v. 5 min before induction of thrombosis by 10 % FeC13 and complete stasis. The control group received PBS ( ) instead of Ir-CPI (■). Each point represents the mean ± SD either for five or six animals.

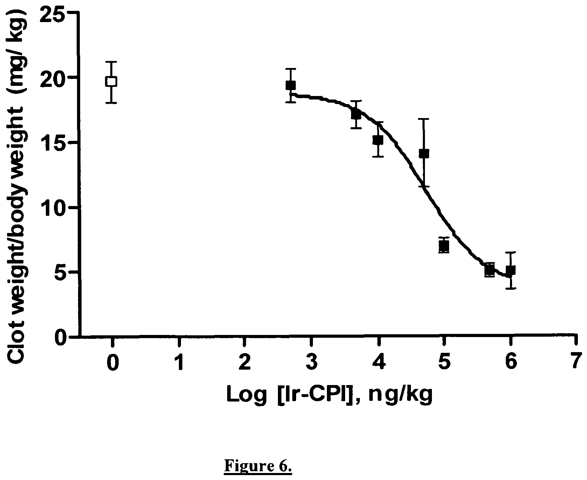

The control group showed 100 % thrombus formation, with a mean thrombus weight of 19.6 ±1.6 mg/kg (n=6). In contrast, prior intravenous administration of Ir-CPI induced a dose-dependent progressive decrease in thrombus formation, with a calculated effective concentration (EC₅₀) at 49.2 µg/kg and with a maximum effect starting at 100 µg/kg (Figure 6).

In order to examine the effect of Ir-CPI on blood coagulation parameters after intravenous administration, the effects of Ir-CPI were then tested on *ex vivo* clotting assays. Table 2 shows that aPTT values were similar in comparison with controls for Ir-CPI EC₅₀ and 100 µg/kg doses whereas aPTT values were statistically higher in comparison with controls for Ir-CPI doses higher than 1 mg/kg, showing a∼1.4-fold increase in the latter case. In contrast, PT was only slightly affected by 1 mg/kg Ir-CPI.

Moreover, this high dose of Ir-CPI had no effect on the fibrinolysis time (Table 2). Finally, the bleeding effect of Ir-CPI was evaluated using a tail-transection model; no statistically significant blood loss was observed 5 min after administration of 1 mg/kg Ir-CPI (Table 2).

**TABLE 2.**

| **Hemostasis and coagulation in Ir-CPI treated rats.** | | | | |
|---|---|---|---|---|
| | PBS | Ir-CPI 0.1 mg/kg | Ir-CPI 1 mg/kg | Heparin 0.5 mg/kg |
| aPTT (s) | 21.0±3.9 | 20.7±3.9 | 29.7±2.7* | ND |
| PT (s) | 38.5±3.4 | 37.9±3.1 | 43.0±1.4* | ND |
| Fibrinolysis time (min) | 52±27 | 66±29 | 51±28 | ND |
| Bleeding effect (O.D. 450 nm) | 0.026±0.006 | 0.067±0.060 | 0.021±0.008 | 0.174±0.091* |

Values are mean ± SD (n = 5, for each test). aPTT, activated partial thromboplastin time; PT, prothrombin time. *P < 0.05, as compared to values observed in the control (PBS) (one-way ANOVA and Student-Newman-Keuls test).

The efficacy of Ir-CPI in inhibiting thrombus formation was also measured on a murine model of venous thrombosis in which complete stasis is induced by ligation of the inferior vena cava (IVC). Five minutes prior to surgery, mice were given an intravenous injection of either Ir-CPI (1 or 10 mg/kg) or the vehicle (PBS) in the caudal vein. Mice were sacrificed 24 hours after thrombosis induction and the thrombosed IVC fragments were harvested and weighed. Results were expressed by dividing the thrombus weight with mouse weight (mg/g) or thrombus length (mg/mm). Data are presented as the mean ± SEM of at least four experiments.

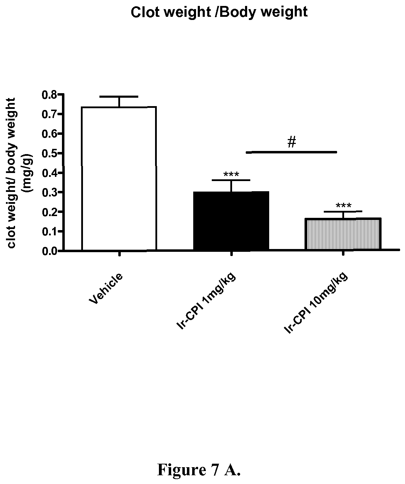

The results showed that Ir-CPI had a dramatic effect on thrombus formation. The clot weight/body weight ratio was significantly reduced in the presence of Ir-CPI, from 0.73 ± 0.05 in the control group to 0.30 ± 0.06 in the group of mice treated preventively by Ir-CPI at a dose of 1 mg/kg (Figure 7A).

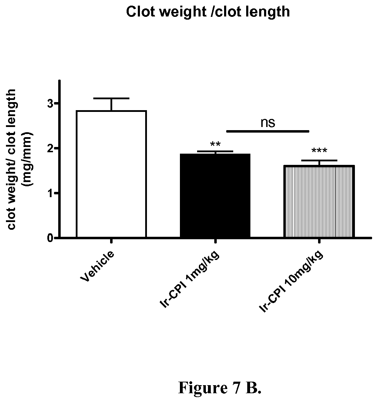

Moreover, the clot weight/clot length ratio also showed that Ir-CPI had a significant effect on the quality of the clot formed. This ratio was 2.8 ± 0.3 for a thrombus formed in the control group whereas it was only 1.9 ± 0.07 in mice treated by a dose of 1 mg/kg of Ir-CPI (Figure 7B).

The effects of Ir-CPI on *ex vivo* clotting assays were also tested. These showed that Ir-CPI at a dose of 1 mg/kg did not modify aPTT, PT and fibrinolysis times. However, aPTT was increased in the presence of Ir-CPI at a dose of 10 mg/kg (22.5 ± 2.0 in the control group vs 41.0 ± 6.0 in the group of Ir-CPI treated mice) whereas PT and fibrinolysis times remain unchanged at this dose.

Finally, the bleeding effect of Ir-CPI was also evaluated by using a tail-transection model. In this case surprisingly, the bleeding time was not affected even after administration of 10 mg/kg Ir-CPI (Table 3).

These results show that Ir-CPI has advantageously an antithrombotic effect *in vivo* without increasing bleeding or impairing blood coagulation parameters.

**TABLE 3.**

| **Hemostasis and coagulation in Ir-CPI treated mice.** | | | | |
|---|---|---|---|---|
| | PBS | Ir-CPI 1 mg/kg | Ir-CPI 10 mg/kg | Heparin 0.5 mg/kg |
| aPTT (s) | 22.5±2.0 | 28.2±6.6 | 41.0±6.0* | 46.8±0.4* |
| PT (s) | 38.7±4.1 | 34.1±2.3 | 36.1±2.5 | 38.4±3.8 |
| Fibrinolysis time (min) | 26±9.7 | 25±11.3 | 21±4.6 | 23±5.0 |
| Bleeding time (min) | 8.6±3.3 | 7.6±4.7 | 14.5±4.8 | >30.0±0.0* |

Values are mean ± SD (n = 5, for each test). aPTT, activated partial thromboplastin time; PT, prothrombin time. *P < 0.05, as compared to values observed in the control (PBS) (one-way ANOVA and Student-Newman-Keuls test).

### Experimental procedures

### Tick material and tick salivary gland extracts

Pathogens free *I. ricinus* ticks were bred and maintained at the Institute of Biology, University of Neuchâtel (Switzerland). Colony founders were initially collected in the field near Neuchâtel and have been maintained on rabbits and mice for over 20 years. Pairs of adult (female and male) ticks were allowed to anchor and feed on rabbits. For preparation of salivary gland extracts, five-day engorged female ticks were dissected under the microscope. Salivary glands were harvested in ice cold phosphate saline buffer. Tissues were then disrupted and homogenized using a dounce. Samples were centrifuged for 5 minutes at 10,000 xg and the supernatants were recovered and stored at -20°C.

Animal care and experimental procedures were carried out in accordance with the Helsinki Declaration (Publication 85-23, revised 1985), local institutional guidelines (laboratory license n° LA 1500474) and the Belgian law of 14th August 1986 as well as the royal decree of 14th of November 1993 on the protection of laboratory animals. Studies were carried out using male Sprague-Dawley OFA rats weighing 250 to 300 g obtained from Harlan (The Netherlands) and 8-week-old NMRI female mice weighing 20 to 25 g, obtained from Elevage Janvier (Le Genest-St-Isle, France).

### Expression and purification of recombinant Ir-CPI in E. coli

The coding region of Ir-CPI cDNA was amplified using a forward primer corresponding to the predicted N-terminal end of mature Ir-CPI (5'-CGCGGATCCGCGGCCAACCACAAAGGTAGAGGG-3'; SEQ.ID.NO.3) and a reverse primer (5'-CCGCTCGAGCGGTTAGACTTTTTTTGCTCTGCATTCC-3'; SEQ.ID.NO.4) corresponding to the C-terminal end of Ir-CPI including the stop codon. BamHI and XhoI restriction enzyme digestion sites were engineered into the 5' and 3' primers, respectively, to enable cloning into the pGEX-6P-1 expression vector (GE Healthcare, Sweden). PCR were performed in a 50 µl reaction volume containing 2.5 U of Taq polymerase (Takara Ex Taq, Takara, Japan), 10 pmoles of specific primers and 2.5 nmoles of each dNTP (Takara) in a standard buffer supplied by the manufacturer (Takara). PCR conditions were as follows: 1 cycle at 95°C for 4 min followed by 30 cycles at 95°C for 30 s/ 58°C for 30 s/ 72°C for 30 s followed by a final extension step at 72°C for 10 min. PCR products were then purified by polyacrylamide gel electrophoresis followed by electroelution. The PCR product was cloned in-frame with GST in the pGEX-6P-1 vector at the BamHI and EcoRI restriction sites and transformed into *E. coli* strain BL21.

Production of the recombinant protein was induced by the addition of IPTG at a final concentration of 1 mM and shaking at 37°C for 2 h. Bacteria were harvested by centrifugation at 4000 xg for 20 min and the pellet was dissolved in PBS. Lysates containing the expressed fusion protein were prepared using a French press. The resulting supernatant, which contained the GST-Ir-CPI fusion protein, was incubated with Glutathione Sepharose High Performance (GE Healthcare, Sweden) and washed. Ir-CPI was released by cleaving with PreScission protease according to the manufacturer's specifications and then purified to homogeneity by gel filtration chromatography using a HiLoad Superdex 75 column (GE Healthcare, Sweden).

### Primary haemostasis

Human blood samples were collected from healthy donors in 0.102 M trisodium citrate tubes (9:1 vol/vol). Global platelet function was measured on a PFA-100 machine (Dade Berhing) with a collagen/epinephrine or collagen/ADP cartridge. The sample (1/10 protein in HBSS and 9/10 citrated whole blood) was aspirated through a capillary under steady high shear rates within 45 min of sample collection.

Platelet plug formation was induced by the presence of the platelet agonists and the high shear rates, and this gradually occluded the aperture. The closure time was considered to be the time required to obtain full occlusion of the aperture. This test measures the time taken for blood platelets to block a membrane coated with collagen and epinephrine (CEPI) or collagen and ADP (CADP) when they are drawn at high shear rates through a fine capillary. This time is called the Closure Time (CT) and is measured in seconds.

The test is therefore a combined measure of platelet adhesion and aggregation. Platelet aggregation was performed using the impedance channel of a Whole Blood Lumi-Aggregometer (Chrono-Log Corp) (Ingerman-Wojenski, 1984). With this method, the electrical impedance between two fine electrodes immersed in the sample increases with platelet coating and aggregation on these electrodes after the addition of an agonist (collagen from Chrono-Log Corp). Platelet aggregation was measured as the maximal/10 minutes change in impedance.

### Anticoagulant Activity

The anticoagulant activities of Ir-CPI were determined by four coagulation tests using a Start8 coagulometer (Diagnostica Stago, France). Human blood samples were collected from healthy donors in 3.8 % trisodium citrate and platelet-poor plasma was obtained by further centrifugation at 4000 g for 10 min.

Activated Partial Thromboplastin Time (aPTT) - Plasma (25 µl) and Ir-CPI (25 µl) were preincubated for 2 min at 37°C. Mixtures were activated for 4 min with 25 µl of Actin FS® (Dade Behring, Germany). Clotting was initiated by adding 50 µl of 25 mM CaCl₂.

Dilute Prothrombin Time (PT) - Plasma (25 µl) and Ir-CPI (25 µl) were preincubated for 2 min at 37°C. Mixtures were activated for 4 min with 25 µl of Innovin® 1/200 (Dade Behring, Germany). The clotting reaction was started by adding 50 µl of 25 mM CaCl₂.

Russel Viper Venom Time - Plasma (25 µl), Hepes buffer (50 µl - Hepes 25 mM, Glycine 2 %, NaCl 145 mM; pH 7.35) and Ir-CPI (25 µl) were preincubated for 2 min at 37°C. Clotting was initiated by the addition of 25 µl of LA 1 (Dade Behring).

Thrombin Time - Plasma (25 µl), Hepes buffer (50 µl) and Ir-CPI (25 µl) were preincubated for 2 min at 37°C. Clotting was initiated by the addition of 25 µl of Thrombin (Diagnostica Stago).

### Determination of clot lysis times

Clot lysis times on platelet-poor plasma (PPP) were determined as described by Zouaoui Boudjeltia *et al.,* (2002). Plasma (100 µl), t-PA (25 µl) and Ir-CPI (100 µl) were preincubated for 2 min at 37°C. Clot formation was started by adding 100 µl (1.5 U/ml) of thrombin. The clot lysis time was measured with a semi- automatic device (Medatronic).

### Thrombin Activity Profiles

Materials: PPP reagent (5 pM TF and 4 µM PL in the final mixture), PPP LOW reagent (1 pM TF and 4 µM PL in the final mixture) and thrombin calibrator were purchased from Synapse BV. For each experiment, a fresh mixture of fluorogenic substrate/calcium chloride buffer solution was prepared as follows: 2275 µl of buffer (Hepes 20 mM, Ph 7.35) containing 60 mg/ml of bovine serum albumin (Sigma) and 240 µl of 1 M calcium chloride were mixed with 60 µl of 100 mM DMSO solution of fluorogenic thrombin substrate (Z-Gly-Gly-Arg-AMC, Bachem). Actin FS® was diluted 25-fold with distilled water.

### Preparation of human plasma

Blood from male healthy volunteers, who were free from medication for at least two weeks, was taken by venipuncture and collected into 0.105 M sodium citrate (9:1 vol/vol). PPP was obtained by centrifugation at room temperature for 10 minutes at 2,500 g and was used immediately after centrifugation.

### Calibrated automated thrombin activity measurement

Thrombin activity measurement was performed using the previously reported CAT procedure (Hemker *et al.,* 2003). Briefly, 80 µl of PPP, 10 µl of PBS or Ir-CPI and 20 µl of PPP reagent, PPP LOW reagent or diluted Actin FS were mixed in a 96-wells microtiter plate (Thermo Immulon 2HB) and were incubated for 5 minutes at 37°C. The coagulation process was triggered by addition of 20 µl of substrate/calcium chloride buffer at 37°C.

A calibration condition was also realized. In this later case, the same protocol as described above using PBS was followed but the activator was replaced by 20 µl of thrombin calibrator. The reaction of fluorogenic thrombin substrate hydrolysis was monitored on a microplate fluorometer Fluoroskan Ascent FL (Thermo Labsystems) with a 390/460 nm filter set (excitation/emission). Fluorescence was measured every 20 s for 60 min. The commercially available Thrombinoscope® software (Synapse BV) processed automatically the acquired data to give thrombin activity profile curves and measurement parameters (lag time and Cmax). Ten Ir-CPI concentrations ranging from 0,001 to 9,077 µM were tested in each experiment in triplicate.

### Assay of the inhibitory effect of Ir-CPI on coagulation factors

The inhibitory activity of Ir-CPI was examined on seven procoagulant serine proteases (plasma kallikrein, FXIIa, FXIa, FIXa, FXa, thrombin and FVIIa) and 2 fibrinolytic serine proteases (t-PA and plasmin). Each serine protease was preincubated with Ir-CPI in a 1:5 molar ratio for 5 min at 37°C, followed by the addition of the appropriate chromogenic substrate (final concentration 0.5 mM). Final concentrations in a total volume of 200 µl in 96-microwell-plates were as follows : kallikrein (3 nM)/S-2302, FXIIa (62.5 nM)/S-2302, FXIa (31.25 nM)/S2366, FIXa (500 nM)/Spectrozyme FIXa, FXa (10 nM)/S-2222, Thrombin (35 nM)/Spectrozyme TH, FT-FVIIa (100 nM)/Spectrozyme FVIIa, t-PA (35 nM)/Spectrozyme t-PA, plasmin (30 nM)/Spectrozyme PL. The kinetics of substrate hydrolysis were measured over 3 min. Chromogenic substrates S-2302, S-2366 and S-2222 were supplied by Chromogenix AB and Spectrozyme FIXa, TH, FVIIa, t-PA, PL were obtained from American Diagnostica Inc.

### Assay of the effects of Ir-CPI on contact system activation in plasma

The effects of Ir-CPI on the activation of the contact system in human plasma were assessed from the generation of activated contact factors (factor XIa, factor XIIa and kallikrein). Human plasma was treated with acid to inactivate plasma serine protease inhibitors and then diluted 1:10 in buffer. Fifty microliters of diluted plasma were incubated with 20 µl of various concentrations of Ir-CPI for 5 min and then activated with 5 µl of aPTT reagent (Actin FS). After 10 min, a chromogenic substrate mixture at a final concentration of 0.5 mM and one or two inhibitors, Corn Trypsin Inhibitor (100 nM) or kallistop (50 µM), were added, and the amidolytic activity of the generated enzyme was determined at 405 nm. Sets of added chromogenic substrate and inhibitors were as follows: S-2366, Kallistop and CTI for factor XIa assay; S-2302 and Kallistop for factor XIIa assay; and S-2302 and CTI for kallikrein assay.

### Assay of the effect of Ir-CPI in a reconstituted system

A reconstitution assay of the kallikrein-kininogen-kinin system was performed using purified coagulation factors (FXIIa and prekallikrein). FXIIa (12.5 nM) was preincubated with Ir-CPI in Hepes buffer for 2 min at 37°C. Prekallikrein (12.5 nM) was added to the mixture, and then prekallikrein activation started. After 10 min, chromogenic substrate S-2302 was added, and the increase in absorbance at 405 nm was recorded over 3 min.

Reconstitution assays of the intrinsic coagulation pathway were performed using purified coagulation factors, factor XI/XIa and factor XII/XIIa. The effect of Ir-CPI on the activation of factor XI by factor XIIa was tested by incubating factor XI (15 nM), factor XIIa (60 nM) and Ir-CPI for 10 min at 37°C. After incubation, substrate S-2366 was added and the increase in absorbance was measured. The effect of Ir-CPI on the activation of factor XII by factor XIa was tested by incubating factor XI (15 nM), factor XIIa (60 nM) and Ir-CPI for 10 min at 37°C. After incubation, substrate S-2302 was added and the increase in absorbance was measured.

Reconstitution assays of the extrinsic coagulation pathway were performed using Actichrome TFPI Activity Assay and recombinant human TFPI according to the manufacturer's specifications (American diagnostica, Stamford).

Reconstitution assay of the fibrinolysis system was performed using purified fibrinolytic factors (t-PA and plasminogen). Plasminogen (500 nM) was preincubated with Ir-CPI for 2 min at 37°C. t-PA (500 nM) was added to the mixture, and plasminogen activation started. After 10 min, Spectrozyme PL chromogenic substrate was added, and the absorbance at 405 nm was measured over 3 min.

### Binding analysis using surface plasmon resonance

The interaction between Ir-CPI and coagulation or fibrinolytic factors was monitored using a BIAcore X instrument (BIAcore AB, Sweden). Ir-CPI (15 µM) was immobilized on the surface of a CM5 sensor chip in 10 mM acetate buffer, pH 5.0, by the amine coupling procedure according to the manufacturer's instructions. 1500 resonance units (RU) of immobilized Ir-CPI were used for the assay. To subtract the non-specific component from the apparent binding response, a blank flow cell was prepared using the same immobilizing procedure without Ir-CPI.

Binding analyses were carried out using HBS buffer (HEPES 10 mM, NaCl 150 mM, EDTA 3 mM; pH 7.4 with 0.005 % surfactant P20) as running buffer at 25°C. 100 µl of each analyte (100 nM) was injected on the sensor chip at a flow rate of 70 µl/min. Association was monitored during an 84-second injection of analyte. Dissociation was monitored for 3 min after return to the running buffer. Regeneration of the sensor chip surface was achieved with a pulse injection (15 µl) of 25 mM NaOH.

The kinetics of interactions between Ir-CPI and the four interacting factors were carried out after a new immobilization of Ir-CPI. The quantity of Ir-CPI immobilized for measurements of kinetics was deliberately maintained at a low level (to approximately 200 RU) to avoid the problems of limitation of the reaction by the process of mass-transport. Independence with respect to differences in flow of the initial rate of connection, measured by linear regression at the start of the kinetics after injections of analytes with increasing flows (30 to 70 µl/min) confirmed that the reactions were not limited by such a process. Interaction kinetics were determined for each analyte, with 6 different concentrations (from 5 nM to 300 nM).

Binding data were analyzed using BIA evaluation software to determine the kinetic constants. The specificity of the reaction was evaluated using the GST protein (about 1000 RU immobilized on the surface of the sensor chip) as negative control.

### Determination of radioactivity of 125I-Ir-CPI in rat blood

¹²⁵I-labeled Ir-CPI was prepared by iodination with [¹²⁵I] sodium iodide in 20 mCi/mg of protein, using IODO-BEADS Iodination Reagent (PIERCE) according to the manufacturer's instructions. Free iodide was removed by extensive gel filtration on Sephadex G10.

The *in vivo* distribution of ¹²⁵I-Ir-CPI in rat blood was evaluated after i.v. administration. Samples containing 10 x 10⁶ cpm were resuspended in 200 µl of PBS and administered to rats. Blood was collected after 3, 20, 40 or 60 h by cardiac puncture in 3.8 % trisodium citrate. Plasma was obtained by centrifugation, and aliquots of 500 µl were placed in glass test tubes. Radioactivity was determined in a gamma counter.

### Ex vivo effect of Ir-CPI on aPTT, PT and fibrinolysis

Ir-CPI was administered i.v. to rats and blood was collected after 5 min by cardiac puncture in 3.8 % trisodium citrate. Platelet-poor plasma was obtained by centrifugation at 4000 g for 10 min. The aPTT, PT and fibrinolysis times were measured using the above-described procedures.

### Complete stasis combined with vessel injury induced venous thrombosis model in the rat

Thrombus formation was induced by a combination of complete stasis and vessel injury by ferric chloride according to the modification of the method described by Peternel *et al.,* (2005). Rats were anesthetized with pentobarbital sodium (70 mg/kg, IP). The abdomen was opened by making an incision along the *linea alba* towards the sternum, followed by exposition of the posterior vena cava. Surgical threads, 1 cm apart, were placed loosely around the vena cava beneath the renal veins and above the bifurcation of the iliac veins to form a snare. Complete stasis was induced in the posterior vena cava by tightening the downstream snare firmly around the posterior vena cava. Simultaneously, a piece of filter paper (0.3 × 0.8 cm) saturated with 10 % w/v ferric chloride solution was applied to the external surface of the posterior vena cava caudally of the ligature for 10 min. Ten min after the removal of the filter paper, the upstream snare was firmly tightened around the posterior vena cava and the rat was then euthanized. The ligated venous segment was excised and the thrombus was removed and immediately weighed after blotting off excess blood.

Results were expressed in milligrams of thrombus per kilogram rat body weight. Ir-CPI (0,5 - 1000 µg/kg) or vehicle were injected in the left femoral vein 5 min prior to the induction of the thrombus formation.

### Experimental venous thrombosis induced by complete stasis in mice

NMRI female mice (20-25 g) were anesthetized with a mixture of ketamine (80 mg/kg) and xylazine (10-16 mg/kg). Aseptic laparotomy was performed; the inferior vena cava (IVC) was isolated and ligated below the renal veins. Major side branches were also ligated. Insides were replaced and the midline incision was closed. Ir-CPI (1 - 10 mg/kg) or vehicle was injected in the caudal vein 5 min prior to surgery. The animals were sacrificed 24 hours after thrombosis induction and thrombosed IVC fragments were harvested and weighed. Results were expressed as thrombus weight over thrombus length (mg/mm) or mouse weight (mg/g).

### Bleeding effect

NMRI female mice (20-25 g Both rat and mouse-tail-transection models were used to evaluate the effect of Ir-CPI on bleeding time. Animals were anesthetized as indicated in previous sections and Ir-CPI was administered i.v. into the vena cava. In the case of rats, the tail was cut 3 mm from the tip after 5 min, and was carefully immersed in 40 ml of distilled water warmed at 37°C. The haemoglobin content of the aqueous solution (absorbance at 540 nm) was used to estimate blood loss. Appropriate controls (i.v. injection of PBS) were run in parallel. In the case of mice, bleeding was monitored by gently absorbing the bead of blood with a filter paper at 15-second intervals without touching the wound. Bleeding was stopped manually if it continued for 30 min.

Data were analyzed by one way ANOVA with a post-hoc Neuman-Keuls multiple comparison test. Statistical significance was assigned for p≤0.05. All experiments were repeated at least three times.

### REFERENCES

1. Dunn JT and Kaplan AP. J Clin Invest. 1982 Sep;70(3):627-31.
2. Renne T and Gailani D. (2007). Expert Rev Cardiovasc Ther. 5(4):733-741.
3. Francischetti, I.M.B., et al., (2004). Thrombosis Haemostasis, 91, 886-898.
4. Kato, N. et al., (2005). Journal of Biochemistry, 38(3), 225-235.
5. Leboulle G, et al., Am J Trop Med Hyg. 2002 Mar;66(3):225-33.
6. Ingerman-Wojenski CM, Silver MJ. 1984;51:154-156.
7. Zouaoui et al. 2002. A new device for measurement of fibrin clot lysis: application to the euglobulin clot lysis time. BMC Biotechnol. 2, 2: 2-8.
8. Mandle RJ Jr and Kaplan AP. Blood. 1979 Oct;54(4):850-62.
9. Hemker HC, et al., Pathophysiol Haemost Thromb 2003;33(1):4-15.
10. Peternel L, et al., Thrombosis Research 2005; 115(6):527-534.

## Claims

1. A pharmaceutical composition consisting of a pharmaceutical carrier or diluent and an effective amount of a polypeptide selected from:
(i) the polypeptide consisting of the sequence SEQ.ID.NO.2, and
(ii) the variants of the polypeptide consisting of the sequence SEQ.ID.NO.2, in which 1 to 10 amino acids are substituted, deleted or added in any combination, and wherein the composition inhibits the activity and/or reduces the content of Factor XIIa, Factor XIa, plasmin and kallikrein.

2. The composition of claim **1** wherein 1 to 5 amino acids are substituted, deleted or added in the variants.

3. The composition of claim **1** wherein 1 or 2 amino acids are substituted, deleted or added in the variants.

4. The composition of any preceding claim wherein the substitutions are among: Ala, Val, Leu and Ile; among Ser and Thr; among Asp and Glu; among Asn and Gln; among Lys and Arg; or among Phe and Tyr.

5. The composition of claim **1** wherein the polypeptide consists of the sequence SEQ.ID.NO.2.

6. The composition of any of the preceding claims wherein the polypeptide is modified by or linked to at least one substitution group selected from the group consisting of amide, acetyl, phosphoryl and/or glycosyl groups.

7. The composition of claim **1** wherein the polypeptide is a variant of the polypeptide consisting of the sequence SEQ.ID.NO.2 in which 1 to 10 amino acids are added, and wherein said amino acids are histidine residues.

8. The composition of claim **1** wherein the polypeptide is a variant of the polypeptide consisting of the sequence SEQ.ID.NO.2 in which 1 to 10 amino acids are added.

9. A polypeptide selected from:
(i) the polypeptide consisting of the sequence SEQ.ID.NO.2, and
(ii) the variants of the polypeptide consisting of the sequence SEQ.ID.NO.2, in which 1 to 10 amino acids are substituted, wherein the substitutions are among: Ala, Val, Leu and He; among Ser and Thr; among Asp and Glu; among Asn and Gln; among Lys and Arg; or among Phe and Tyr.

10. The polypeptide of claim **9** consisting of the sequence SEQ.ID.NO.2.

11. The polypeptide of any of claims **9** to **10** modified by or linked to at least one substitution group selected from the group consisting of amide, acetyl, phosphoryl and/or glycosyl groups.

12. A polynucleotide sequence encoding the polypeptide of any of claims **9** to **11** or consisting of SEQ.ID.NO:1.

13. A vector comprising the polynucleotide of claim **12.**

14. A cell comprising the vector according to claim **13.**

15. A pharmaceutical composition comprising a pharmaceutical carrier and the polypeptide of any of claims **9** to **11.**

16. The use of the polypeptide of any of claims **9** to **11** or the polynucleotide of claim 12 as a research reagent or material for the development of treatments and diagnostic tools specific to human disease, wherein said use is not methods for treatment of the human or animal body by surgery or therapy nor diagnostic methods practised on the human or animal body.

17. The pharmaceutical composition of claim **15** for use in the treatment and/or the prevention of thrombosis and/or cardiovascular diseases, optionally while preserving clotting balance.

18. The pharmaceutical composition for use of claim **17** that is for use in inhibiting the intrinsic coagulation pathway.

19. The pharmaceutical composition for use of claim **17** or claim **18** wherein the disease is selected from the group consisting of thromboembolism, stroke, myocardial infarction, cerebrovascular diseases, cerebral ischemia, pulmonary embolism, renal vein thrombosis and hepatic vein thrombosis.

## Patentansprüche

1. Pharmazeutische Zusammensetzung, bestehend aus einem pharmazeutischen Trägerstoff oder Verdünnungsmittel und einer wirksamen Menge eines Polypeptids, ausgewählt aus:
(i) dem Polypeptid bestehend aus der Sequenz SEQ.ID.NO.2, und
(ii) den Varianten des Polypeptids, bestehend aus der Sequenz SEQ.ID.NO.2, in der 1 bis 10 Aminosäuren in jeder Kombination substituiert, deletiert oder zugegeben sind,
und wobei die Zusammensetzung die Aktivität von Faktor XIIa, Faktor XIa, Plasmin und Kallikrein hemmt und/oder den Gehalt davon reduziert.

2. Zusammensetzung nach Anspruch **1,** wobei 1 bis 5 Aminosäuren in den Varianten substituiert, deletiert oder zugegeben sind.

3. Zusammensetzung nach Anspruch **1,** wobei 1 oder 2 Aminosäuren in den Varianten substituiert, deletiert oder zugegeben sind.

4. Zusammensetzung nach einem der vorhergehenden Ansprüche, wobei die Substitutionen unter Folgenden sind: Ala, Val, Leu und Ile; unter Ser und Thr; unter Asp und Glu; unter Asn und Gln; unter Lys und Arg; oder unter Phe und Tyr.

5. Zusammensetzung nach Anspruch **1,** wobei das Polypeptid aus der Sequenz SEQ.ID.NO.2 besteht.

6. Zusammensetzung nach einem der vorhergehenden Ansprüche, wobei das Polypeptid durch mindestens eine Substitutionsgruppe modifiziert oder damit verbunden ist, ausgewählt aus der Gruppe, bestehend aus Amid-, Acetyl-, Phosphoryl- und/oder Glycosylgruppen.

7. Zusammensetzung nach Anspruch **1,** wobei das Polypeptid eine Variante des Polypeptids ist, bestehend aus der Sequenz SEQ.ID.NO.2, in der 1 bis 10 Polypeptide zugegeben sind, und wobei die Aminosäuren Histidinreste sind.

8. Zusammensetzung nach Anspruch **1,** wobei das Polypeptid eine Variante des Polypeptids ist, bestehend aus der Sequenz SEQ.ID.NO.2, wobei 1 bis 10 Polypeptide zugegeben sind.

9. Polypeptid, ausgewählt aus:
(i) dem Polypeptid, bestehend aus der Sequenz SEQ.ID.NO.2, und
(ii) den Varianten des Polypeptids, bestehend aus der Sequenz SEQ.ID.NO.2, in der 1 bis 10 Aminosäuren substituiert sind, wobei die Substitutionen unter Folgenden sind: Ala, Val, Leu und Ile; unter Ser und Thr; unter Asp und Glu; unter Asn und Gln; unter Lys und Arg; oder unter Phe und Tyr.

10. Polypeptid nach Anspruch **9,** bestehend aus der Sequenz SEQ.ID.NO.2.

11. Polypeptid nach einem der Ansprüche **9** bis **10,** das durch mindestens eine Substitutionsgruppe modifiziert oder damit verbunden ist, ausgewählt aus der Gruppe, bestehend aus Amid-, Acetyl-, Phosphoryl- und/oder Glycosylgruppen.

12. Polynucleotidsequenz, die das Polypeptid nach einem der Ansprüche **9** bis **11** codiert oder aus SEQ.ID.NO.1 besteht.

13. Vektor, umfassend das Polynukleotid nach Anspruch **12.**

14. Zelle, umfassend den Vektor nach Anspruch **13.**

15. Pharmazeutische Zusammensetzung, umfassend einen pharmazeutischen Trägerstoff und das Polypeptid nach einem der Ansprüche **9** bis **11.**

16. Verwendung des Polypeptids nach einem der Ansprüche **9** bis **11** oder des Polypeptids nach Anspruch **12** als ein Forschungsreagenz oder Material für die Entwicklung von Behandlungen und Diagnosewerkzeugen, die spezifisch für menschliche Erkrankungen sind, wobei die Verwendung weder Verfahren zur Behandlung des menschlichen oder tierischen Körpers durch Chirurgie oder Therapie, noch Diagnoseverfahren die am menschlichen oder tierischen Körper durchgeführt werden darstellen.

17. Pharmazeutische Zusammensetzung nach Anspruch **15** zur Verwendung bei der Behandlung und/oder Prävention von Thrombose und/oder Herz-Kreislauf-Erkrankungen, optional unter Beibehaltung der Koagulationsbilanz.

18. Pharmazeutische Zusammensetzung zur Verwendung nach Anspruch **17,** die zur Verwendung bei der Hemmung des intrinsischen Koagulationswegs ist.

19. Pharmazeutische Zusammensetzung zur Verwendung nach Anspruch **17** oder Anspruch **18,** wobei die Erkrankung ausgewählt ist aus der Gruppe, bestehend aus Thromoboembolie, Schlaganfall, Myokardinfarkt, zerebrovaskuläre Erkrankungen, zerebrale Ischämie, Lungenembolie, Nierenvenenthrombose und Lebervenenthrombose.

## Revendications

1. Une composition pharmaceutique consistant en un support ou diluant pharmaceutique et une quantité efficace d'un polypeptide sélectionné parmi :
(i) le polypeptide consistant en la séquence SEQ.ID.NO.2, et
(ii) les variants du polypeptide consistant en la séquence SEQ.ID.NO.2, dans lequel 1 à 10 acides aminés sont substitués, supprimés ou ajoutés suivant n'importe quelle combinaison,
et dans laquelle la composition inhibe l'activité et/ou réduit la teneur en Facteur XIIa, Facteur XIa, plasmine et kallicréine.

2. La composition selon la revendication **1** dans laquelle 1 à 5 acides aminés sont substitués, supprimés ou ajoutés dans les variants.

3. La composition selon la revendication **1** dans laquelle 1 ou 2 acides aminés sont substitués, supprimés ou ajoutés dans les variants.

4. La composition selon l'une quelconque des revendications précédentes dans laquelle les substitutions sont parmi : Ala, Val, Leu et Ile ; parmi Ser et Thr ; parmi Asp et Glu ; parmi Asn et Gln ; parmi Lys et Arg ; ou parmi Phe et Tyr.

5. La composition selon la revendication **1** dans laquelle le polypeptide consiste en la séquence SEQ.ID.NO.2.

6. La composition selon l'une quelconque des revendications précédentes dans laquelle le polypeptide est modifié par ou lié à au moins un groupe de substitution sélectionné dans le groupe consistant en les groupes amide, acétyle, phosphoryle et/ou glycosyle.

7. La composition selon la revendication **1** dans laquelle le polypeptide est un variant du polypeptide consistant en la séquence SEQ.ID.NO.2 dans lequel 1 à 10 acides aminés sont ajoutés, et dans lequel lesdits acides aminés sont des résidus histidine.

8. La composition selon la revendication **1** dans laquelle le polypeptide est un variant du polypeptide consistant en la séquence SEQ.ID.NO.2 dans lequel 1 à 10 acides aminés sont ajoutés.

9. Un polypeptide sélectionné parmi :
(i) le polypeptide consistant en la séquence SEQ.ID.NO.2, et
(ii) les variants du polypeptide consistant en la séquence SEQ.ID.NO.2, dans lequel 1 à 10 acides aminés sont substitués, dans lequel les substitutions sont parmi : Ala, Val, Leu et Ile ; parmi Ser et Thr ; parmi Asp et Glu ; parmi Asn et Gln ; parmi Lys et Arg ; ou parmi Phe et Tyr.

10. Le polypeptide selon la revendication **9** consistant en la séquence SEQ.ID.NO.2.

11. Le polypeptide selon l'une quelconque des revendications **9** à **10** modifié par ou lié à au moins un groupe de substitution sélectionné dans le groupe consistant en les groupes amide, acétyle, phosphoryle et/ou glycosyle.

12. Une séquence polynucléotidique codant pour le polypeptide de l'une quelconque des revendications **9** à **11** ou consistant en la SEQ.ID.NO.1.

13. Un vecteur comprenant le polynucléotide de la revendication **12.**

14. Une cellule comprenant le vecteur de la revendication **13.**

15. Une composition pharmaceutique comprenant un support pharmaceutique et le polypeptide de l'une quelconque des revendications **9** à **11.**

16. L'utilisation du polypeptide de l'une quelconque des revendications **9** à **11** ou du polynucléotide de la revendication **12** en tant que réactif de recherche ou matériel pour le développement de traitements et d'outils de diagnostic spécifique à la maladie humaine, dans laquelle ladite utilisation n'est pas des méthodes de traitement chirurgical ou thérapeutique du corps humain ou animal ni des méthodes de diagnostic appliquées au corps humain ou animal.

17. La composition pharmaceutique selon la revendication **15** pour son utilisation dans le traitement et/ou la prévention de la thrombose et/ou des maladies cardiovasculaires, optionnellement tout en préservant l'équilibre de la coagulation.

18. La composition pharmaceutique pour son utilisation selon la revendication **17** pour son utilisation dans l'inhibition de la voie de coagulation intrinsèque.

19. La composition pharmaceutique pour son utilisation selon la revendication **17** ou la revendication **18** dans laquelle la maladie est sélectionnée dans le groupe consistant en la thromboembolie, l'accident vasculaire cérébral, l'infarctus du myocarde, les maladies vasculaires cérébrales, l'ischémie cérébrale, l'embolisme pulmonaire, la thrombose veineuse rénale et la thrombose vénale hépatique.
